# EUROPEAN PATENT APPLICATION

(11) **EP 3 738 972 A1**
(43) Date of publication of application: **18.11.2020**
(21) Application number: 19174101.6
(22) Date of filing: 13.05.2019
(51) Int. Cl.: C07K 14/005

(54) **PARVOLYSINE**

(71) Applicant: Deutsches Krebsforschungszentrum, 69120 Heidelberg (DE)
(72) Inventor: The designation of the inventor has not yet been filed
(74) Representative: Altmann Stößel Dick Patentanwälte PartG mbB

(57) **Abstract**

The present invention relates to a composition comprising at least one polynucleotide comprising (i) an expression construct encoding an NS1 polypeptide of a parvovirus and (ii) an expression construct encoding a viroporin and to a composition comprising an NS1 polypeptide of a parvovirus and a viroporin, wherein the mass ratio of NS1 polypeptide to viroporin is at most 100. Further, the present invention relates to said compositions for use as a medicine and for use in the treatment of inappropriate cellular proliferation, and to a method for identifying a subject susceptible to treatment of inappropriate cellular proliferation with a composition of the invention.

SEQUENCE LISTING

<110>  Deutsches Krebsforschungszentrum

<120>  Parvolysine

<130>  DK15384EP

<160>  15

<170>  PatentIn version 3.3

<210>  1
<211>  437
<212>  PRT
<213>  Artificial

<220>
<223>  truncated NS1 polypeptide

<400>  1

Lys Thr Asn Phe Leu Lys Glu Gly Glu Arg His Leu Val Ser Lys Leu
1               5                   10                  15

Tyr Thr Asp Asp Met Arg Pro Glu Thr Val Glu Thr Thr Val Thr Thr
            20                  25                  30

Ala Gln Glu Thr Lys Arg Gly Arg Ile Gln Thr Lys Lys Glu Val Ser
        35                  40                  45

Ile Lys Thr Thr Leu Lys Glu Leu Val His Lys Arg Val Thr Ser Pro
    50                  55                  60

Glu Asp Trp Met Met Met Gln Pro Asp Ser Tyr Ile Glu Met Met Ala
65                  70                  75                  80

Gln Pro Gly Gly Glu Asn Leu Leu Lys Asn Thr Leu Glu Ile Cys Thr
                85                  90                  95

Leu Thr Leu Ala Arg Thr Lys Thr Ala Phe Asp Leu Ile Leu Glu Lys
            100                 105                 110

Ala Glu Thr Ser Lys Leu Thr Asn Phe Ser Leu Pro Asp Thr Arg Thr
        115                 120                 125

Cys Arg Ile Phe Ala Phe His Gly Trp Asn Tyr Val Lys Val Cys His
    130                 135                 140

Ala Ile Cys Cys Val Leu Asn Arg Gln Gly Gly Lys Arg Asn Thr Val
145                 150                 155                 160

Leu Phe His Gly Pro Ala Ser Thr Gly Lys Ser Ile Ile Ala Gln Ala

165                 170                 175

Ile Ala Gln Ala Val Gly Asn Val Gly Cys Tyr Asn Ala Ala Asn Val
        180                 185                 190

Asn Phe Pro Phe Asn Asp Cys Thr Asn Lys Asn Leu Ile Trp Val Glu
        195                 200                 205

Glu Ala Gly Asn Phe Gly Gln Gln Val Asn Gln Phe Lys Ala Ile Cys
    210                 215                 220

Ser Gly Gln Thr Ile Arg Ile Asp Gln Lys Gly Lys Gly Ser Lys Gln
225                 230                 235                 240

Ile Glu Pro Thr Pro Val Ile Met Thr Thr Asn Glu Asn Ile Thr Val
                245                 250                 255

Val Arg Ile Gly Cys Glu Glu Arg Pro Glu His Thr Gln Pro Ile Arg
        260                 265                 270

Asp Arg Met Leu Asn Ile His Leu Thr His Thr Leu Pro Gly Asp Phe
        275                 280                 285

Gly Leu Val Asp Lys Asn Glu Trp Pro Met Ile Cys Ala Trp Leu Val
    290                 295                 300

Lys Asn Gly Tyr Gln Ser Thr Met Ala Ser Tyr Cys Ala Lys Trp Gly
305                 310                 315                 320

Lys Val Pro Asp Trp Ser Glu Asn Trp Ala Glu Pro Lys Val Pro Thr
                325                 330                 335

Pro Ile Asn Leu Leu Gly Ser Ala Arg Ser Pro Phe Thr Thr Pro Lys
        340                 345                 350

Ser Thr Pro Leu Ser Gln Asn Tyr Ala Leu Thr Pro Leu Ala Ser Asp
        355                 360                 365

Leu Glu Asp Leu Ala Leu Glu Pro Trp Ser Thr Pro Asn Thr Pro Val
    370                 375                 380

Ala Gly Thr Ala Glu Thr Gln Asn Thr Gly Glu Ala Gly Ser Lys Ala
385                 390                 395                 400

Cys Gln Asp Gly Gln Leu Ser Pro Thr Trp Ser Glu Ile Glu Glu Asp
                405                 410                 415

Leu Arg Ala Cys Phe Gly Ala Glu Pro Leu Lys Lys Asp Phe Ser Glu
            420                 425                 430

Pro Leu Asn Leu Asp
        435

<210>  2
<211>  58
<212>  PRT
<213>  Minute virus of mice

<400>  2

Met Ala Pro Ala Asn Leu Thr Ala Glu Thr Leu Ser Thr Gln Leu Gln
1               5                   10                  15

Glu Leu Asn Glu Gln Leu Thr Ala Leu Glu Ala Leu Gly Val Gly Ala
            20                  25                  30

Leu Ala Gly Val Gly Leu Val Phe Leu Leu Gly Leu Met Ile Ile Lys
        35                  40                  45

Arg Ile Ile Asp Ser Trp Val Thr Ala Gly
    50                  55

<210>  3
<211>  142
<212>  PRT
<213>  Artificial

<220>
<223>  unique sequence in VP1

<400>  3

Met Ala Pro Pro Ala Lys Arg Ala Lys Arg Gly Trp Val Pro Pro Gly
1               5                   10                  15

Tyr Lys Tyr Leu Gly Pro Gly Asn Ser Leu Asp Gln Gly Glu Pro Thr
            20                  25                  30

Asn Pro Ser Asp Ala Ala Ala Lys Glu His Asp Glu Ala Tyr Asp Gln
        35                  40                  45

Tyr Ile Lys Ser Gly Lys Asn Pro Tyr Leu Tyr Phe Ser Ala Ala Asp
    50                  55                  60

Gln Arg Phe Ile Asp Gln Thr Lys Asp Ala Lys Asp Trp Gly Gly Lys
65                  70                  75                  80

Val Gly His Tyr Phe Phe Arg Thr Lys Arg Ala Phe Ala Pro Lys Leu
                85                  90                  95

Ala Thr Asp Ser Glu Pro Gly Thr Ser Gly Val Ser Arg Ala Gly Lys
            100                 105                 110

Arg Thr Arg Pro Pro Ala Tyr Ile Phe Ile Asn Gln Ala Arg Ala Lys
        115                 120                 125

Lys Lys Leu Thr Ser Ser Ala Ala Gln Gln Ser Ser Gln Thr
    130                 135                 140

<210>  4
<211>  10
<212>  PRT
<213>  Artificial

<220>
<223>  myc-tag

<400>  4

Glu Gln Lys Leu Ile Ser Glu Glu Asp Leu
1               5                   10

<210>  5
<211>  177
<212>  DNA
<213>  Minute virus of mice

<400>  5
atggcaccag ccaacctgac agcggaaacg ctgtccactc agctgcaaga gttgaacgag      60

cagctgacgg ccctggaggc tctgggggtg ggggctctgg cgggggtggg gttggtgttt     120

ctactgggtc ttatgataat caaacgcatt atagattctt gggtgacggc tgggtag        177

<210>  6
<211>  1314
<212>  DNA
<213>  Artificial

<220>
<223>  truncated NS1 polypeptide encoding sequence

<400>  6
aaaactaact ttttaaaaga aggcgagcgc catctagtga gcaaactata cactgatgac      60

atgcggccag aaacggttga aaccacagta accactgcgc aggaaactaa gcgcggcaga     120

attcaaacta aaaaagaagt ttctattaaa actacactta aagagctggt gcataaaaga     180

gtaacctcac cagaggactg gatgatgatg cagccagaca gttacattga aatgatggct     240

caaccaggtg gagaaaacct gctgaaaaat acgctagaga tttgtacact aactctagcc     300

agaaccaaaa cagcatttga cttaatttta gaaaaagctg aaaccagcaa actaaccaac     360

ttttcactgc ctgacacaag aacctgcaga atttttgctt ttcatggctg gaactatgtt     420

aaagtttgcc atgctatttg ctgtgtttta aacagacaag gaggcaaaag aaatactgtt 480

ttatttcatg gaccagccag cacaggcaaa tctattattg cacaagccat agcacaagca 540

gttggcaatg ttggttgcta taatgcagcc aatgtaaact ttccatttaa tgactgtacc 600

aacaagaact tgatttgggt agaagaagct ggtaactttg gacagcaagt aaaccagttt 660

aaagccattt gctctggtca aactattcgc attgatcaaa aaggaaaagg cagcaaacag 720

attgaaccaa caccagtcat catgaccaca aatgagaaca ttacagtggt cagaataggc 780

tgcgaagaaa gaccagaaca cactcaacca atcagagaca gaatgcttaa cattcatcta 840

acacatacct tgcctggtga ctttggtttg gttgacaaaa atgaatggcc catgatttgt 900

gcttggttgg taaagaatgg ttaccaatct accatggcaa gctactgtgc taaatggggc 960

aaagttcctg attggtcaga aaactgggcg gagccaaagg tgccaactcc tataaattta 1020

ctaggttcgg cacgctcacc attcacgaca ccgaaaagta cgcctctcag ccagaactat 1080

gcactaactc cacttgcatc ggatctcgag gacctggctt tagagccttg gagcacacca 1140

aatactcctg ttgcgggcac tgcagaaacc cagaacactg gggaagctgg ttccaaagcc 1200

tgccaagatg gtcaactgag cccaacttgg tcagagatcg aggaggattt gagagcgtgc 1260

ttcggtgcgg aaccgttgaa gaaagacttc agcgagccgc tgaacttgga ctaa 1314

<210> 7
<211> 22
<212> DNA
<213> Artificial

<220>
<223> MVMp Fig. 4

<400> 7
accatgagtg atggcaccag cc 22

<210> 8
<211> 8
<212> PRT
<213> Artificial

<220>
<223> ORF1 VP2 Fig.4

<400> 8

Thr Met Ser Asp Gly Thr Ser Gln
1               5

<210> 9
<211> 5
<212> PRT
<213> Artificial

<220>
<223>  ORF2 SAT Fig. 4

<400>  9

Val Met Ala Pro Ala
1               5


<210>  10
<211>  28
<212>  DNA
<213>  Artificial

<220>
<223>  wt Fig. 6

<400>  10
cagcaaagca gtcaaaccat gagtgatg                                          28


<210>  11
<211>  28
<212>  DNA
<213>  Artificial

<220>
<223>  myc-wt Fig. 6

<400>  11
cagtaaagca gtcaaaccat gagtgatg                                          28


<210>  12
<211>  21
<212>  DNA
<213>  Artificial

<220>
<223>  SUPER Fig. 6

<400>  12
cagtaaagca gtcaaaccat g                                                 21


<210>  13
<211>  21
<212>  DNA
<213>  Artificial

<220>
<223>  myc-SUPER Fig. 6

<400>  13
cagtaaagca gtcaaaccat g                                                 21


<210>  14
<211>  198
<212>  PRT
<213>  minute virus of mice

<400>  14

Met Ala Pro Pro Ala Lys Arg Ala Lys Arg Gly Trp Val Pro Pro Gly
1               5                   10                  15

Tyr Lys Tyr Leu Gly Pro Gly Asn Ser Leu Asp Gln Gly Glu Pro Thr
            20                  25                  30

Asn Pro Ser Asp Ala Ala Ala Lys Glu His Asp Glu Ala Tyr Asp Gln
        35                  40                  45

Tyr Ile Lys Ser Gly Lys Asn Pro Tyr Leu Tyr Phe Ser Ala Ala Asp
    50                  55                  60

Gln Arg Phe Ile Asp Gln Thr Lys Asp Ala Lys Asp Trp Gly Gly Lys
65                  70                  75                  80

Val Gly His Tyr Phe Phe Arg Thr Lys Arg Ala Phe Ala Pro Lys Leu
                85                  90                  95

Ala Thr Asp Ser Glu Pro Gly Thr Ser Gly Val Ser Arg Ala Gly Lys
            100                 105                 110

Arg Thr Arg Pro Pro Ala Tyr Ile Phe Ile Asn Gln Ala Arg Ala Lys
        115                 120                 125

Lys Lys Leu Thr Ser Ser Ala Ala Gln Gln Ser Ser Gln Thr Met Ser
    130                 135                 140

Asp Gly Thr Ser Gln Pro Asp Ser Gly Asn Ala Val His Ser Ala Ala
145                 150                 155                 160

Arg Val Glu Arg Ala Ala Asp Gly Pro Gly Gly Ser Gly Gly Gly Gly
                165                 170                 175

Ser Gly Gly Gly Gly Val Gly Val Ser Thr Gly Ser Tyr Asp Asn Gln
            180                 185                 190

Thr His Tyr Arg Phe Leu
        195

<210>  15
<211>  197
<212>  PRT
<213>  Parvovirus H1

<400>  15

Met Ala Pro Pro Ala Lys Arg Ala Lys Arg Gly Trp Val Pro Pro Gly
1               5                   10                  15

Tyr Lys Tyr Leu Gly Pro Gly Asn Ser Leu Asp Gln Gly Glu Pro Thr
            20                  25                  30

Asn Pro Ser Asp Ala Ala Ala Lys Glu His Asp Glu Ala Tyr Asp Gln
        35                  40                  45

Tyr Ile Lys Ser Gly Lys Asn Pro Tyr Leu Tyr Phe Ser Pro Ala Asp
    50                  55                  60

Gln Arg Phe Ile Asp Gln Thr Lys Asp Ala Lys Asp Trp Gly Gly Lys
65                  70                  75                  80

Val Gly His Tyr Phe Phe Arg Thr Lys Arg Ala Phe Ala Pro Lys Leu
                85                  90                  95

Ser Thr Asp Ser Glu Pro Gly Thr Ser Gly Val Ser Arg Pro Gly Lys
            100                 105                 110

Arg Thr Lys Pro Pro Ala His Ile Phe Val Asn Gln Ala Arg Ala Lys
        115                 120                 125

Lys Lys Arg Ala Ser Leu Ala Ala Gln Gln Arg Thr Leu Thr Met Ser
    130                 135                 140

Asp Gly Thr Glu Thr Asn Gln Pro Asp Thr Gly Ile Ala Asn Ala Arg
145                 150                 155                 160

Val Glu Arg Ser Ala Asp Gly Gly Gly Ser Ser Gly Gly Gly Gly Ser
                165                 170                 175

Gly Gly Gly Gly Ile Gly Val Ser Thr Gly Thr Tyr Asp Asn Gln Thr
            180                 185                 190

Thr Tyr Lys Phe Leu
        195

## Description

The present invention relates to a composition comprising at least one polynucleotide comprising (i) an expression construct encoding an NS1 polypeptide of a parvovirus and (ii) an expression construct encoding a viroporin and to a composition comprising an NS1 polypeptide of a parvovirus and a viroporin, wherein the mass ratio of NS1 polypeptide to viroporin is at most 100. Further, the present invention relates to said compositions for use as a medicine and for use in the treatment of inappropriate cellular proliferation, and to a method for identifying a subject susceptible to treatment of inappropriate cellular proliferation with a composition of the invention.

Protoparvoviruses (PV) are well known for their intrinsic oncotropism and oncolytic activities - essential features for a successful virotherapy of cancer. This was recently established in a first phase I/IIa clinical trial treating patients with glioblastoma multiforma and a variety of compassionate treatments using propagation-competent H-1PV (Geletneky et al. (2012), BMC cancer 12:99; Geletneky et al. (2017), J Am Soc Gene Ther 25:2620; Angelova et al. (2017), Viruses 9(12):382). Besides strategies involving replication-competent protoparvoviruses, replication-incompetent armed parvoviral vectors for the therapy of various cancer entities were established combining PV oncolytic activities with immune-stimulating agents (Rommelaere et al. (2010), Cytokine Growth Factor Rev. 21(2-3):185; Marchini et al. (2016), Viruses 8(1):9). Besides these parvoviral agents, a major interest consists in the use of PV-oncotoxicity/oncolysis administrated by non-viral delivery systems (Nuesch et al. (2014), Adv Exp Med Biol 818:99-124).

The large non-structural PV protein NS1 was identified as the major toxic polypeptide interfering with multiple cellular pathways and processes in neoplastically transformed cells which eventually becomes deleterious for NS1-expressing cells and finally leads to cell death. In order to achieve all these tasks during viral replication, NS1 functioning is tightly regulated through expression, posttranslational modification, and subcellular trafficking (Nuesch (2006). Regulation of non-structural protein functions by differential synthesis, modification and trafficking, p. 275-286. In Bloom MEC, S.F.; Linden, R.M.; Parrish, C.R.; and Kerr, J.R. (ed.), Parvoviruses. Hodder Arnold, London). Interestingly, many NS1 functions are dependent on factors which are activated or up-regulated upon transformation, accounting for the intrinsic oncotoxic activity of the polypeptide (Angelova (2015), Front Bioeng Biotechnol 3:55). Among the regulatory mechanisms for NS1 toxicity, the PDK-1 (phospho-inositide-dependent kinase 1)/PKC/PKB (Akt1) signaling cascade, which is frequently induced upon transformation, appears to play a crucial role. On the other hand, this pathway was shown to modulate targets of PVs in order to achieve permissiveness for these agents (Nuesch & Rommelaere (2007), PNAS 104:12482-12487; Bär et al. (2015), PLoS pathogens 11:e1004703). Although PV NS1 is capable of killing neoplastically transformed cells while sparing their normal counterparts, induction of cell death appears to be a rather uncontrolled process due to the multiple functions and tasks of NS1 during viral infection, ranging from enzymatic activities to interactions with cell proteins, interference with replication/repair, transcription/translation machineries to intracellular transport and signaling cascades (Nuesch et al. (2014), loc. cit., Nuesch et al. (2012), Clinical Cancer Res 18:3516-3523).

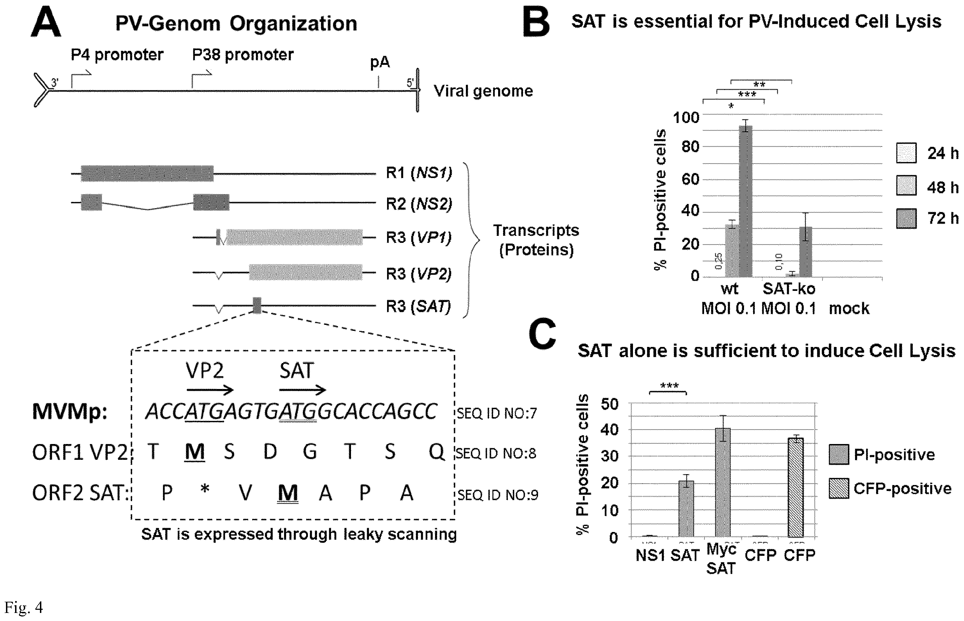

The PV SAT protein was discovered as a "late" regulatory protein of unknown function by Zadori et al. ((2005), J Virol 79:13129-13138). This ER-resident protein appeared to induce an ER-stress response and in consequence cell death, independent of other viral proteins (Meszaros (2017), J Virol. 91(16):e00627-17). SAT typically comprises <100 amino acids and it contains a highly hydrophobic transmembrane domain. SAT-knockout PV produce a strongly reduced lytic phenotype compared to the wild type counterparts (Fig. 4, Bretscher, Ph D Thesis; University of Heidelberg).

There is, nonetheless, a need in the art to provide improved means and methods for cancer treatment. In particular, there is a need to provide means and methods avoiding at least in part the drawbacks of the prior art as discussed above.

This problem is solved by the compositions, uses, and methods with the features of the independent claims. Preferred embodiments, which might be realized in an isolated fashion or in any arbitrary combination are listed in the dependent claims.

Accordingly, the present invention relates to a composition comprising at least one polynucleotide comprising (i) an expression construct encoding an NS1 polypeptide of a parvovirus and (ii) an expression construct encoding a viroporin..

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, as used in the following, the terms "preferably", "more preferably", "most preferably", "particularly", "more particularly", "specifically", "more specifically" or similar terms are used in conjunction with optional features, without restricting further possibilities. Thus, features introduced by these terms are optional features and are not intended to restrict the scope of the claims in any way. The invention may, as the skilled person will recognize, be performed by using alternative features. Similarly, features introduced by "in an embodiment" or similar expressions are intended to be optional features, without any restriction regarding further embodiments of the invention, without any restrictions regarding the scope of the invention and without any restriction regarding the possibility of combining the features introduced in such way with other optional or non-optional features of the invention.

As used herein, the term "standard conditions", if not otherwise noted, relates to IUPAC standard ambient temperature and pressure (SATP) conditions, i.e. preferably, a temperature of 25°C and an absolute pressure of 100 kPa; also preferably, standard conditions include a pH of 7. Moreover, if not otherwise indicated, the term "about" relates to the indicated value with the commonly accepted technical precision in the relevant field, preferably relates to the indicated value ± 20%, more preferably ± 10%, most preferably ± 5%. Further, the term "essentially" indicates that deviations having influence on the indicated result or use are absent, i.e. potential deviations do not cause the indicated result to deviate by more than ± 20%, more preferably ± 10%, most preferably ± 5%. Thus, "consisting essentially of' means including the components specified but excluding other components except for materials present as impurities, unavoidable materials present as a result of processes used to provide the components, and components added for a purpose other than achieving the technical effect of the invention. For example, a composition defined using the phrase "consisting essentially of' encompasses any known acceptable additive, excipient, diluent, carrier, and the like. Preferably, a composition consisting essentially of a set of components will comprise less than 5% by weight, more preferably less than 3% by weight, even more preferably less than 1%, most preferably less than 0.1% by weight of non-specified component(s). In the context of nucleic acid sequences, the term "essentially identical" indicates a %identity value of at least 80%, preferably at least 90%, more preferably at least 98%, most preferably at least 99%. As will be understood, the term essentially identical includes 100% identity. The aforesaid applies to the term "essentially complementary" mutatis mutandis.

As used herein, the term "composition" relates to a composition comprising the indicated compounds. Preferably, the composition is a mixture of compounds comprising at least one polynucleotide as specified herein and at least one carrier. The carrier(s) must be acceptable in the sense of being compatible with the other ingredients of the composition and being not deleterious to a potential recipient thereof. The carrier employed may be, for example, either a solid, a gel or a liquid. Exemplary of solid carriers are lactose, terra alba, sucrose, talc, gelatin, agar, pectin, acacia, magnesium stearate, stearic acid and the like. Exemplary of liquid carriers are phosphate buffered saline solution, syrup, oil such as peanut oil and olive oil, water, emulsions, various types of wetting agents, sterile solutions and the like. Similarly, the carrier or diluent may include time delay material well known to the art, such as glyceryl mono-stearate or glyceryl distearate alone or with a wax. Suitable carriers comprise those mentioned above and others well known in the art. The carrier(s) is/are selected so as not to affect the biological activity of the composition. Examples of such diluents are distilled water, physiological saline, Ringer's solutions, dextrose solution, and Hank's solution.

Preferably, the composition is a pharmaceutical composition; thus, preferably, the carrier is a pharmaceutically acceptable carrier. In addition, the pharmaceutical composition or formulation may also include other carriers, adjuvants, or nontoxic, nontherapeutic, non-immunogenic stabilizers and the like. The polynucleotide of the present invention can be formulated as a pharmaceutically acceptable salt. Acceptable salts comprise acetate, methylester, HCl, sulfate, chloride and the like. The pharmaceutical compositions are, preferably, administered topically or systemically. Suitable routes of administration conventionally used for drug administration are oral, intravenous, or parenteral administration as well as inhalation. However, depending on the nature and mode of action of a compound, the pharmaceutical compositions may be administered by other routes as well, in particular as specified elsewhere herein. Moreover, the polynucleotide can be administered in combination with other drugs either in a common pharmaceutical composition or as separated pharmaceutical compositions wherein said separated pharmaceutical compositions may be provided in form of a kit of parts. The composition is, preferably, administered in conventional dosage forms prepared by combining the compounds with standard pharmaceutical carriers according to conventional procedures. These procedures may involve mixing, granulating and compressing or dissolving the ingredients as appropriate to the desired preparation. It will be appreciated that the form and character of the pharmaceutically acceptable carrier or diluent is dictated by the amount of active ingredient with which it is to be combined, the route of administration and other well-known variables.

A therapeutically effective dose refers to an amount of the polynucleotide to be used in a pharmaceutical composition of the present invention which prevents, ameliorates or treats the symptoms accompanying a disease or condition referred to in this specification. Therapeutic efficacy and toxicity of such compounds can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors; preferably in accordance with any one of the above described methods. As is well known in the medical arts, dosages for any one patient depend upon many factors, which may include the patient's size, body surface area, age, the particular compound to be administered, sex, time and route of administration, general health, and other drugs being administered concurrently. Progress can be monitored by periodic assessment. A typical dose can be, for example, in the range of 1 to 1000 µg; however, doses below or above this exemplary range are envisioned, especially considering the aforementioned factors. Generally, the regimen as a regular administration of the pharmaceutical composition should be in the range of 1 µg to 10 mg units per day. If the regimen is a continuous infusion, it should also be in the range of 1 µg to 1 mg units per kilogram of body weight per minute, respectively. Progress can be monitored by periodic assessment. However, depending on the subject and the mode of administration, the quantity of substance administration may vary over a wide range to provide from about 0.01 mg per kg body mass to about 10 mg per kg body mass. The pharmaceutical compositions and formulations referred to herein are administered at least once in order to treat or ameliorate or prevent a disease or condition recited in this specification. However, the said pharmaceutical compositions may be administered more than one time, for example from one to four times daily up to a non-limited number of days. Preferably, treating or preventing a disease or condition with a compound recited in this specification consists of a single administration of said compound.

Specific pharmaceutical compositions are prepared in a manner well known in the pharmaceutical art and comprise at least one active compound referred to herein above in admixture or otherwise associated with a pharmaceutically acceptable carrier or diluent. For making those specific pharmaceutical compositions, the active compound(s) will usually be mixed with a carrier or the diluent, or enclosed or encapsulated in a capsule, sachet, cachet, paper or other suitable containers or vehicles. The resulting formulations are to be adopted to the mode of administration, i.e. in the forms of tablets, capsules, suppositories, solutions, suspensions or the like. Dosage recommendations shall be indicated in the prescribers or user's instructions in order to anticipate dose adjustments depending on the considered recipient.

Preferably, the at least one polynucleotide is comprised as an isolated polynucleotide in the composition. More preferably, the polynucleotide is comprised in the composition as a complex, i.e. in a non-covalent association with a further compound. Preferably, said further compound is a compound mediating entry of a polynucleotide into a host cell; such compounds are known in the art. Preferably, the compound mediating entry of a polynucleotide into a host cell is a transfection agent like calcium phosphate, polyethyleneimines, or an amphiphilic transfection reagent. More preferably, the compound mediating entry of a polynucleotide into a host cell is or comprises a viral capsid, wherein, preferably, said viral capsid comprises said polynucleotide, preferably as part of or replacing the viral genome. Methods for packaging DNA into viral capsids or virus-like particles are known in the art.

The term "polynucleotide" is known to the skilled person. As used herein, the term includes nucleic acid molecules comprising or consisting of a nucleic acid sequence or nucleic acid sequences as specified herein. The polynucleotide of the present invention shall be provided, preferably, either as an isolated polynucleotide (i.e. isolated from its natural context) or in genetically modified form. The polynucleotide, preferably, is DNA, including cDNA, or is RNA. The term encompasses single as well as double stranded polynucleotides. Preferably, the polynucleotide is a chimeric molecule, i.e., preferably, comprises at least one nucleic acid sequence, preferably of at least 20 bp, more preferably at least 100 bp, heterologous to the residual nucleic acid sequences. Moreover, preferably, comprised are also chemically modified polynucleotides including naturally occurring modified polynucleotides such as glycosylated or methylated polynucleotides or artificial modified one such as biotinylated polynucleotides.

As used herein, the term polynucleotide, preferably, includes variants of the specifically indicated polynucleotides. More preferably, the term polynucleotide relates to the specific polynucleotides indicated. It is to be understood, however, that a polypeptide having a specific amino acid sequence may be encoded by a variety of polynucleotides, due to the degeneration of the genetic code. The skilled person knows how to select a polynucleotide encoding a polypeptide having a specific amino acid sequence and also knows how to optimize the codons used in the polynucleotide according to the codon usage of the organism used for expressing said polynucleotide. Thus, the term "polynucleotide variant", as used herein, relates to a variant of a polynucleotide related to herein comprising a nucleic acid sequence characterized in that the sequence can be derived from the aforementioned specific nucleic acid sequence by at least one nucleotide substitution, addition and/or deletion, wherein the polynucleotide variant shall have the activity as specified for the specific polynucleotide, i.e. shall encode a polypeptide according to the present invention. Preferably, said polynucleotide variant is an ortholog, a paralog or another homolog of the specific polynucleotide. Also preferably, said polynucleotide variant is a naturally occurring allele of the specific polynucleotide. Polynucleotide variants also encompass polynucleotides comprising a nucleic acid sequence which is capable of hybridizing to the aforementioned specific polynucleotides, preferably, under stringent hybridization conditions. These stringent conditions are known to the skilled worker and can be found in Current Protocols in Molecular Biology, John Wiley & Sons, N. Y. (1989), 6.3.1-6.3.6. A preferred example for stringent hybridization conditions are hybridization conditions in 6x sodium chloride/sodium citrate (= SSC) at approximately 45°C, followed by one or more wash steps in 0.2x SSC, 0.1% SDS at 50 to 65°C. The skilled worker knows that these hybridization conditions differ depending on the type of nucleic acid and, for example when organic solvents are present, with regard to the temperature and concentration of the buffer. For example, under "standard hybridization conditions" the temperature differs depending on the type of nucleic acid between 42°C and 58°C in aqueous buffer with a concentration of 0.1x to 5x SSC (pH 7.2). If organic solvent is present in the abovementioned buffer, for example 50% formamide, the temperature under standard conditions is approximately 42°C. The hybridization conditions for DNA:DNA hybrids are preferably for example 0.1x SSC and 20°C to 45°C, preferably between 30°C and 45°C. The hybridization conditions for DNA:RNA hybrids are preferably, for example, 0.1x SSC and 30°C to 55°C, preferably between 45°C and 55°C. The abovementioned hybridization temperatures are determined for example for a nucleic acid with approximately 100 bp (= base pairs) in length and a G + C content of 50% in the absence of formamide. The skilled worker knows how to determine the hybridization conditions required by referring to textbooks such as the textbook mentioned above, or the following textbooks: Sambrook et al., "Molecular Cloning", Cold Spring Harbor Laboratory, 1989; Hames and Higgins (Ed.) 1985, "Nucleic Acids Hybridization: A Practical Approach", IRL Press at Oxford University Press, Oxford; Brown (Ed.) 1991, "Essential Molecular Biology: A Practical Approach", IRL Press at Oxford University Press, Oxford. Alternatively, polynucleotide variants are obtainable by PCR-based techniques such as mixed oligonucleotide primer- based amplification of DNA, i.e. using degenerated primers against conserved domains of a polypeptide of the present invention. Conserved domains of a polypeptide may be identified by a sequence comparison of the nucleic acid sequence of the polynucleotide or the amino acid sequence of the polypeptide of the present invention with sequences of other organisms. As a template, DNA or cDNA from viruses, bacteria, fungi, plants, or animals, preferably from a virus, may be used. Further, variants include polynucleotides comprising nucleic acid sequences which are at least 70%, at least 75%, at least 80%, at least 85%, at least 90%, at least 95%, at least 98% or at least 99% identical to the specifically indicated nucleic acid sequences. Moreover, also encompassed are polynucleotides which comprise nucleic acid sequences encoding amino acid sequences which are at least 70%, at least 75%, at least 80%, at least 85%, at least 90%, at least 95%, at least 98% or at least 99% identical to the amino acid sequences specifically indicated. The percent identity values are, preferably, calculated over the entire amino acid or nucleic acid sequence region. A series of programs based on a variety of algorithms is available to the skilled worker for comparing different sequences. In this context, the algorithms of Needleman and Wunsch or Smith and Waterman give particularly reliable results. To carry out the sequence alignments, the program PileUp (Feng & Doolittle (1987), J. Mol. Evolution. 25:351-360; Higgins et al. (1989), CABIOS, 5:151-153) or the programs Gap and BestFit

(Needleman and Wunsch (1970), J. Mol. Biol. 48:443-453 and Smith and Waterman (1981), Adv. Appl. Math. 2: 482-489), which are part of the GCG software packet (Genetics Computer Group, 575 Science Drive, Madison, Wisconsin, USA 53711 (1991)), are to be used. The sequence identity values recited above in percent (%) are to be determined, preferably, using the program GAP over the entire sequence region with the following settings: Gap Weight: 50, Length Weight: 3, Average Match: 10.000 and Average Mismatch: 0.000, which, unless otherwise specified, shall always be used as standard settings for sequence alignments.

A polynucleotide comprising a fragment of any of the specifically indicated nucleic acid sequences is also encompassed as a variant polynucleotide of the present invention. The fragment shall still encode an immunogenic polypeptide which still has the activity as specified. Accordingly, the immunogenic polypeptide encoded may comprise or consist of the domains of the immunogenic polypeptide of the present invention conferring the said biological activity. A fragment as meant herein, preferably, comprises at least 50, at least 100, at least 250 or at least 500 consecutive nucleotides of any one of the specific nucleic acid sequences or encodes an amino acid sequence comprising at least 20, at least 30, at least 50, at least 80, at least 100 or at least 150 consecutive amino acids of any one of the specific amino acid sequences.

The polynucleotides of the present invention either consist, essentially consist of, or comprise the aforementioned nucleic acid sequences. Thus, they may contain further nucleic acid sequences as well. Specifically, the polynucleotides of the present invention may encode fusion proteins wherein one partner of the fusion protein is an immunogenic polypeptide being encoded by a nucleic acid sequence recited above. Such fusion proteins may comprise as additional part polypeptides for monitoring expression (e.g., green, yellow, blue or red fluorescent proteins, alkaline phosphatase and the like), so called "tags" which may serve as a detectable marker or as an auxiliary measure for purification purposes, and/or scaffold polypeptides such as thioredoxin, as described herein above. Tags for the different purposes are well known in the art and are described elsewhere herein.

The term "expression construct", as used herein, relates to a polynucleotide operatively linked to at least one expression control sequence causing transcription of the nucleic acid sequence comprised in said polynucleotide to occur, preferably in eukaryotic cells or isolated fractions thereof, preferably into a translatable mRNA or into a viral genome. Methods for providing expression constructs are known to the skilled person, who is also aware that expression of a given construct may be context dependent and may in particular depend on the type of cell (i.e. prokaryotic, archeal, or eukaryotic cell, plant or animal cells, specific cells type, and the like). Preferably, the expression construct is a eukaryotic expression construct, more preferably a mammalian expression construct, still more preferably a cancer cell expression construct. Preferably, the expression construct is mammalian cancer cell specific, i.e. causes expression in at least one type of mammalian cancer cell at least 10fold, preferably at least 20fold, higher than in a mammalian non-cancer cell. Regulatory elements ensuring expression in eukaryotic cells, preferably mammalian cells, are well known in the art. They, preferably, comprise regulatory sequences ensuring initiation of transcription and, optionally, poly-A signals ensuring termination of transcription and stabilization of the transcript. Additional regulatory elements may include transcriptional as well as translational enhancers. Preferably, the aforesaid at least one expression control sequence is an expression control sequence of a single-stranded DNA virus, more preferably of a Parvovirus as described elsewhere herein, most preferably of a parvovirus strain MVMp MV. Preferably, the expression construct as specified herein is comprised in the genome of a Parvovirus. Thus, preferably, the at least one polynucleotide as specified herein further encodes all structural polypeptides of a parvovirus. More preferably, the at least one polynucleotide comprises a parvovirus genome comprising an expression construct encoding a viroporin. Also preferably, the at least one polynucleotide is comprised in a parvovirus particle. Method of packaging polynucleotides into parvoviral particles are known in the art, e.g. reviewed in Cornelis et al. (2004), J Gene Med. 6 Suppl 1:S193-202.

The composition as referred to herein comprises at least one polynucleotide comprising (i) an expression construct encoding an NS1 polypeptide of a parvovirus and (ii) an expression construct encoding a viroporin. As will be understood, the two aforesaid expression constructs may be comprised on one polynucleotide, i.e. the same polynucleotide; they may, however, also be comprised by two or more polynucleotides, i.e. by non-identical polynucleotides. Preferably, the expression construct encoding a viroporin comprises, preferably consists of, the sequence of SEQ ID NO:5 or a sequence at least 70% identical to said sequence. Also preferably, the expression construct encoding a viroporin encodes an RNA comprising the open reading frame encoding said viroporin as the only open reading frame encoding more than 40 continuous amino acids. More preferably, the first start codon of the RNA encoded by the expression construct encoding said viroporin is the start codon for the open reading frame encoding said viroporin. Preferably, the expression construct encoding an NS1 polypeptide comprises, preferably consists of the sequence of SEQ ID NO:6 or a sequence at least 70% identical to said sequence, more preferably comprises, preferably consists of the sequence of SEQ ID NO:6. Also preferably, at least the NS1 polypeptide and the viroporin are encoded by the same expression construct; more preferably, the start codon for the open reading frame encoding said viroporin is the first start codon downstream of an internal ribosome entry site of said expression construct encoding an NS1 polypeptide. Also preferably, the at least one polynucleotide further comprises an expression construct encoding a VP1u polypeptide as specified herein below. Also preferably, the expression construct encoding an NS1 polypeptide comprises a parvovirus genome.

The term "parvovirus" is known to the skilled person as relating to a member of the Parvoviridae family of small, linear and non-segmented single-stranded DNA viruses. Preferably, the parvovirus is a member of the genus Erythroparvovirus, more preferably is a primate Erythroparvovirus 1, also known as Parvovirus B19. Most preferably, the parvovirus is a minute virus of mice (MVM), preferably strain MVMp. Preferably, the parvovirus has a nucleic acid sequence as specified in Genbank Acc. No. J02275.1 or a sequence at least 70% identical thereto. More preferably, the parvovirus has a nucleic acid sequence as specified in Genbank Acc. No. J02275.1.

As used herein, the term "NS1 polypeptide" of a parvovirus relates to the large non-structural protein 1 of a parvovirus, having the biological activity of inducing cell death in neoplastically transformed mammalian cells. The activity of inducing cell death by an NS1 polypeptide can be established by methods known in the art, preferably in an MTT assay, which is known in the art. The MTT assay is a colorimetric assay for measuring cell metabolic activity. It is based on the ability of nicotinamide adenine dinucleotide phosphate (NADPH)-dependent cellular oxidoreductase enzymes to reduce the tetrazolium dye MTT to its insoluble formazan, which has a purple color. Preferably, said activity is measured in ELISA plates by a determining a decrease of the optical Density (OD) at 570 in treated cells compared to control (untreated) cells. Preferably, the NS1 polypeptide comprises amino acids 235 to 672 of an NS1 polypeptide, i.e. has an amino acid sequence as shown in SEQ ID NO:1 or a sequence at least 70% identical thereto; more preferably, has an amino acid sequence as shown in SEQ ID NO:1. Even more preferably, the NS1 polypeptide comprises, more preferably consists of, the amino acid sequence of Genbank Acc. No. AAA67109.1 or a sequence at least 70% identical thereto; more preferably, the NS1 polypeptide comprises, more preferably consists of, the amino acid sequence of Genbank Acc. No. AAA67109.1.

The term "viroporin", as used herein, relates to a member of a group of small and usually hydrophobic multifunctional viral proteins known under this name to the skilled person, having the biological activity of forming ion channels in biological membranes. The activity of a polypeptide of forming ion channels is preferably determined by staining cells comprising said polypeptide with a dye normal cells are not permeable to, preferably with a dye comprising propidium ions, more preferably with propidium iodide, and comparing the amount of dye entering the cells to control cells not comprising said polypeptide. Preferably, the viroporin is a parvoviral small alternatively translated (SAT) polypeptide. Preferably, the viroporin consists of from 30 to 150 amino acids, more preferably of from 40 to 125 amino acids, most preferably of from 50 to 90 amino acids. Preferably, the viroporin is encoded on a parvovirus genome. Preferably, the viroporin comprises, more preferably consists of an amino acid sequence encoded by nucleotides 2801 to 2977 of Genbank Acc. No. J02275.1, i.e. preferably an amino acid sequence as shown in SEQ ID NO:2 or a sequence at least 70% identical thereto; more preferably, an amino acid sequence as shown in SEQ ID NO:2. Preferably, the viroporin is a fusion polypeptide comprising a SAT polypeptide of a parvovirus, preferably as specified herein above, and a stabilizing peptide. Preferably, the stabilizing polypeptide has the biological activity of delaying degradation of a viroporin in a mammalian cell, preferably by at least a factor of 2. More preferably, the stabilizing polypeptide is a short peptide of from 5 to 25 amino acids, more preferably of from 7 to 12 amino acids, preferably comprising at least 2, more preferably at least 3, most preferably at least 4 acidic amino acids. Preferably, the stabilizing polypeptide is a myc-tag, more preferably an N-terminal myc-tag, most preferably an N-terminal myc-tag comprising, preferably consisting of the sequence of SEQ ID NO:4. Also preferably, the stabilizing peptide comprises, preferably consists of, an amino acid sequence encoded by any one of SEQ ID NOs: 10 to 13.

The term "VP1 polypeptide", as used herein, relates to a parvovirus minor capsid polypeptide known to the skilled person. In accordance, the term "VPlu polypeptide" relates to a polypeptide comprising, preferably consisting of the N-terminal extension comprised in parvovirus VP1 compared to the parvovirus VP2 polypeptide. Thus, preferably, the VP1u polypeptide comprises, preferably consists of, the sequence unique in VP1 compared to VP2. Preferably, the VP1u polypeptide comprises, preferably consists of, the amino acid sequence shown in SEQ ID NO:3 or a sequence at least 70% identical thereto; more preferably, an amino acid sequence as shown in SEQ ID NO:3. In accordance with the above, the VPlu polypeptide may preferably also be a VP1 polypeptide or a truncated version thereof comprising the sequence as specified above.

Preferably, variants of the aforesaid polypeptides are also included. As used herein, the term "polypeptide variant" relates to any chemical molecule comprising at least one polypeptide or fusion polypeptide as specified elsewhere herein, having the indicated activity, but differing in primary structure from said polypeptide or fusion polypeptide indicated above. Thus, the polypeptide variant, preferably, is a mutein having the indicated biological activity. Preferably, the polypeptide variant comprises a peptide having an amino acid sequence corresponding to an amino acid sequence of 5 to 200, more preferably 6 to 100, even more preferably 7 to 50, or, most preferably, 8 to 30 consecutive amino acids comprised in a polypeptide as specified above. Moreover, it is to be understood that a polypeptide variant as referred to in accordance with the present invention shall preferably have an amino acid sequence which differs due to at least one amino acid substitution, deletion and/or addition, wherein the amino acid sequence of the variant is still, preferably, at least 70%, 80%, 85%, 90%, 92%, 95%, 97%, 98%, or 99% identical with the amino acid sequence of the specific polypeptide. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the sequence it is compared to for optimal alignment. The percentage is calculated by determining, preferably over the whole length of the polypeptide, the number of positions at which the identical amino acid residue occurs in both sequences to yield the number of matched positions, dividing the number of matched positions by the total number of positions in the window of comparison and multiplying the result by 100 to yield the percentage of sequence identity. Preferably, the comparison window comprises a complete sequence as specified herein. Optimal alignment of sequences for comparison may be conducted by the local homology algorithm of Smith and Waterman (1981), by the homology alignment algorithm of Needleman and Wunsch (1970), by the search for similarity method of Pearson and Lipman (1988), by computerized implementations of these algorithms

(GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Polypeptide variants referred to herein may be allelic variants or any other species specific homologs, paralogs, or orthologs. Moreover, the polypeptide variants referred to herein preferably include fragments of the specific polypeptides or the aforementioned types of polypeptide variants as long as these fragments and/or variants have the biological activity as referred to above. Such fragments may be or be derived from, e.g., degradation products or splice variants of the polypeptides. Further included are variants which differ due to posttranslational modifications such as phosphorylation, glycosylation, ubiquitinylation, sumoylation, or myristylation, by including non-natural amino acids, and/or by being peptidomimetics.

Advantageously, it was found in the work underlying the present invention that expression of a combination of an NS1 polypeptide and a SAT polypeptide causes highly efficient lysis of tumor cells. This effect can be further corroborated by co-expressing a VPlu sequence.

The definitions made above apply mutatis mutandis to the following. Additional definitions and explanations made further below also apply for all embodiments described in this specification mutatis mutandis.

The present invention also relates to a composition comprising an NS1 polypeptide of a parvovirus and a viroporin, wherein the mass ratio of NS1 polypeptide to viroporin is at most 100, preferably at most 10, more preferably at most 1.

The definition of the term "composition" as provided herein above applies to the composition comprising an NS1 polypeptide of a parvovirus and a viroporin mutatis mutandis.

The present invention further relates to a composition as specified herein for use as a medicine or for use in the treatment of inappropriate cellular proliferation.

The term "inappropriate cellular proliferation", as used herein, relates to an abnormal proliferation of body cells in a subject; as a consequence, an imbalance of cellular composition of a body tissue, of a body fluid and/or tumor formation may ensue. Inappropriate cellular proliferation may be induced by an infectious agent, preferably a virus, more preferably an oncogenic virus, more preferably Epstein-Barr virus, a hepatitis virus, Human T-lymphotropic virus 1, a papillomavirus, Human herpesvirus 8, or a human papillomavirus (HPV). Inappropriate cellular proliferations may, however, also be induced by chemical compounds, e.g. a carcinogen, or endogenously, e.g. caused by spontaneous mutation. Preferably, inappropriate cellular proliferation is non-transient. Also preferably, inappropriate cellular proliferation is malignant, i.e. does threaten health or life of a subject; thus, preferably, the malignant inappropriate cellular proliferation is cancer. The term "cancer", as used herein, relates to a disease of an animal, including man, characterized by uncontrolled growth by a group of body cells ("cancer cells"). This uncontrolled growth may be accompanied by intrusion into and destruction of surrounding tissue and possibly spread of cancer cells to other locations in the body. Preferably, also included by the term cancer is a relapse. Thus, preferably, the cancer is a solid cancer, a metastasis, or a relapse thereof.

Preferably, the cancer is selected from the list consisting of acute lymphoblastic leukemia, acute myeloid leukemia, adrenocortical carcinoma, aids-related lymphoma, anal cancer, appendix cancer, astrocytoma, atypical teratoid, basal cell carcinoma, bile duct cancer, bladder cancer, brain stem glioma, breast cancer, burkitt lymphoma, carcinoid tumor, cerebellar astrocytoma, cervical cancer, chordoma, chronic lymphocytic leukemia, chronic myelogenous leukemia, colon cancer, colorectal cancer, craniopharyngioma, endometrial cancer, ependymoblastoma, ependymoma, esophageal cancer, extracranial germ cell tumor, extragonadal germ cell tumor, extrahepatic bile duct cancer, fibrosarcoma, gallbladder cancer, gastric cancer, gastrointestinal stromal tumor, gestational trophoblastic tumor, hairy cell leukemia, head and neck cancer, hepatocellular cancer, hodgkin lymphoma, hypopharyngeal cancer, hypothalamic and visual pathway glioma, intraocular melanoma, kaposi sarcoma, laryngeal cancer, medulloblastoma, medulloepithelioma, melanoma, merkel cell carcinoma, mesothelioma, mouth cancer, multiple endocrine neoplasia syndrome, multiple myeloma, mycosis fungoides, nasal cavity and paranasal sinus cancer, nasopharyngeal cancer, neuroblastoma, non-hodgkin lymphoma, non-small cell lung cancer, oral cancer, oropharyngeal cancer, osteosarcoma, ovarian cancer, ovarian epithelial cancer, ovarian germ cell tumor, ovarian low malignant potential tumor, pancreatic cancer, papillomatosis, paranasal sinus and nasal cavity cancer, parathyroid cancer, penile cancer, pharyngeal cancer, pheochromocytoma, pituitary tumor, pleuropulmonary blastoma, primary central nervous system lymphoma, prostate cancer, rectal cancer, renal cell cancer, retinoblastoma, rhabdomyosarcoma, salivary gland cancer, sézary syndrome, small cell lung cancer, small intestine cancer, soft tissue sarcoma, squamous cell carcinoma, squamous neck cancer, testicular cancer, throat cancer, thymic carcinoma, thymoma, thyroid cancer, urethral cancer, uterine sarcoma, vaginal cancer, vulvar cancer, waldenström macroglobulinemia, and wilms tumor. More preferably, the cancer is glioblastoma multiforme (GBM), pancreatic cancer, preferably pancreatic ductal carcinoma (PDAC), colon carcinoma, melanoma, and/or liver cancer, preferably hepatocellular cancer (HCC).

The terms "treating" and "treatment" refer to an amelioration of the diseases or disorders referred to herein or the symptoms accompanied therewith to a significant extent. Said treating as used herein also includes an entire restoration of health with respect to the diseases or disorders referred to herein. It is to be understood that treating, as the term is used herein, may not be effective in all subjects to be treated. However, the term shall require that, preferably, a statistically significant portion of subjects suffering from a disease or disorder referred to herein can be successfully treated. Whether a portion is statistically significant can be determined without further ado by the person skilled in the art using various well known statistic evaluation tools, e.g., determination of confidence intervals, p-value determination, Student's t-test, Mann-Whitney test etc.. Preferred confidence intervals are at least 90%, at least 95%, at least 97%, at least 98% or at least 99 %. The p-values are, preferably, 0.1, 0.05, 0.01, 0.005, or 0.0001. Preferably, the treatment shall be effective for at least 10%, at least 20% at least 50%at least 60%, at least 70%, at least 80%, or at least 90% of the subjects of a given cohort or population. Preferably, treating cancer is reducing tumor burden in a subject. As will be understood by the skilled person, effectiveness of treatment of e.g. cancer is dependent on a variety of factors including, e.g. cancer stage and cancer type. Preferably, treating causes inappropriately proliferating cells, preferably neoplastic cells, more preferably cancer cells, to enter into a proliferation arrest and/or to lyse. Thus, preferably, treating has the effect of causing a tumor to stop growing, more preferably to cause regression of a tumor, more preferably of causing a tumor to resolve.

The present invention further relates to a use of a composition as specified herein for the manufacture of a medicament, preferably a medicament for the treatment of cancer.

The present invention also relates to a method for treating inappropriate cellular proliferation in a subject, comprising
i) contacting said subject with a composition according to any one of claims 1 to 20, and
ii) thereby treating inappropriate cellular proliferation.

The method of treating of the present invention, preferably, is an in vivo method. Preferably, one or more of said steps may be performed by automated equipment. Moreover, the method may comprise steps in addition to those explicitly mentioned above. For example, further steps may relate, e.g., to diagnosing inappropriate cellular proliferation before step a), or administration of further treatment before, during, or after step a). As will be understood by the skilled person, the aforesaid further treatment may in particular be virotherapy, chemotherapy, radiotherapy, targeted therapy, and/or immunotherapy.

The term "virotherapy", as used herein, relates to an administration of a replication-competent virus to cancer cells. Administration of said virus may be topical or systemic. Preferably, said virus administered is an oncolytic virus, more preferably a measles virus or, preferably, a parvovirus. As will be understood by the skilled person, a composition as specified herein, preferably a polynucleotide as specified herein, may be comprised in the oncolytic virus; thus, the oncolytic virus may e.g. be a parvovirus armed with a polynucleotide as specified.

As used herein, the term "chemotherapy" relates to treatment of a subject with an antineoplastic drug. Preferably, chemotherapy is a treatment including alkylating agents (e.g. cyclophosphamide), platinum (e.g. carboplatin), antimetabolites (e.g. 5-Fluorouracil), anthracyclines (e.g. doxorubicin, epirubicin, idarubicin, or daunorubicin), topoisomerase II inhibitors (e.g. etoposide, irinotecan, topotecan, camptothecin, or VP16), anaplastic lymphoma kinase (ALK)-inhibitors (e.g. Crizotinib or AP26130), aurora kinase inhibitors (e.g. N-[4-[4-(4-Methylpiperazin-1-yl)-6-[(5-methyl-1H-pyrazol-3-yl)amino]pyrimidin-2-yl]sulfanylphenyl]cyclopropanecarboxamide (VX-680)), or Iodine131-1-(3-iodobenzyl)guanidine (therapeutic metaiodobenzylguanidine), or histone deacetylase (HDAC) inhibitors, alone or any suitable combination thereof. It is to be understood that chemotherapy, preferably, relates to a complete cycle of treatment, i.e. a series of several (e.g. four, six, or eight) doses of antineoplastic drug or drugs applied to a subject, which may be separated by several days or weeks without such application.

The terms "radiation therapy" and "radiotherapy" are known to the skilled artisan. The term relates to the use of ionizing radiation to treat or control cancer.

The term "targeted therapy", as used herein, relates to application to a patient a chemical substance known to block growth of cancer cells by interfering with specific molecules known to be necessary for tumorigenesis or cancer or cancer cell growth. Examples known to the skilled artisan are small molecules like, e.g. PARP-inhibitors (e.g. Iniparib), antiangiogenic agents (e.g. Bevacizumab, Ramucirumab, Ziv-aflibercept), signalling inhibitors (e.g. cetuximab or panitumumab), or kinase inhibitors (e.g. Regorafenib).

The term "immunotherapy" as used herein relates to the treatment of cancer by modulation of the immune response of a subject. Said modulation may be inducing, enhancing, or suppressing said immune response, e.g. by administration of at least one cytokine, and/or of at least one antibody specifically recognizing cancer cells. The term "cell based immunotherapy" relates to a cancer therapy comprising application of immune cells, e.g. T-cells, preferably tumor-specific NK cells, to a subject.

The term "subject", as used herein, relates to an animal, preferably a vertebrate, more preferably a mammal, in particular to livestock like cattle, horse, pig, sheep, and goat, or to a laboratory animal like a rat, mouse, and guinea pig. Most preferably, the subject is a human. Preferably, the subject is known or suspected to suffer from inappropriate cellular proliferation, more preferably from cancer.

Furthermore, the present invention relates to a method for identifying a subject susceptible to treatment of inappropriate cellular proliferation with a composition according to the present invention, comprising
a) contacting a test sample of inappropriately proliferating cells of said subject with a composition according to the present invention,
b) determining viability of cells comprised in the sample of a) after said contacting, and
c) thereby identifying a subject susceptible to treatment.

The method for identifying of the present invention, preferably, is an in vitro method. Preferably, one or more of said steps may be performed by automated equipment. Moreover, the method may comprise steps in addition to those explicitly mentioned above. For example, further steps may relate, e.g., obtaining a sample of inappropriately proliferating cells for step a), or incubation of the cells of step a) before performing step b).

The term "test sample", as used herein, refers to a sample to be used for the method for identifying a subject susceptible to treatment of inappropriate cellular proliferation with a composition according to the present invention. Said test sample is a biological sample comprising a plurality of living cells, preferably inappropriately proliferating cells. Preferred biological samples to be used in the method of the present invention are samples from body fluids, preferably, blood, plasma, serum, lymph, or urine, or samples derived, e.g., by biopsy, from cells, tissues or organs, preferably tissue suspected to include or essentially consist of inappropriately proliferating cells. Techniques for obtaining the aforementioned different types of biological samples are well known in the art. For example, blood samples may be obtained by blood taking while tissue or organ samples are to be obtained, e.g., by biopsy.

In view of the above, the following embodiments are particularly envisaged:
Embodiment 1: A composition comprising at least one polynucleotide comprising (i) an expression construct encoding an NS1 polypeptide of a parvovirus and (ii) an expression construct encoding a viroporin.
Embodiment 2: The composition of embodiment 1, wherein said viroporin comprises a SAT polypeptide of a parvovirus.
Embodiment 3: The composition of embodiment 1 or 2, wherein said viroporin is a fusion polypeptide comprising a SAT polypeptide of a parvovirus and a stabilizing peptide, preferably a myc-tag, more preferably an N-terminal myc-tag.
Embodiment 4: The composition of any one of embodiments 1 to 3, wherein said viroporin comprises the amino acid sequence of SEQ ID NO:2 or a sequence at least 70% identical thereto.
Embodiment 5: The composition of any one of embodiments 1 to 4, wherein said expression construct encoding a viroporin encodes an RNA comprising the open reading frame encoding said viroporin as the only open reading frame encoding more than 40 continuous amino acids.
Embodiment 6: The composition of any one of embodiments 1 to 5, wherein the first start codon of the RNA encoded by the expression construct encoding said viroporin is the start codon for the open reading frame encoding said viroporin.
Embodiment 7: The composition of any one of embodiments 1 to 6, wherein at least said NS1 polypeptide and said viroporin are encoded by the same expression construct. Embodiment 8: The composition of any one of embodiments 1 to 7, wherein the start codon for the open reading frame encoding said viroporin is the first start codon downstream of an internal ribosome entry site of said expression construct encoding an NS1 polypeptide. Embodiment 9: The composition of any one of embodiments 1 to 8, wherein said expression construct encoding a viroporin comprises the sequence of SEQ ID NO:5 or a sequence at least 70% identical to said sequence.
Embodiment 10: The composition of any one of embodiments 1 to 9, wherein said NS1 polypeptide comprises at least the amino acids corresponding to amino acids 235 to 672 of the NS1 polypeptide of parvovirus strain MVMp (SEQ ID NO:1) or a sequence at least 70% identical therto..
Embodiment 11: The composition of any one of embodiments 1 to 10, wherein said expression construct encoding an NS1 polypeptide comprises the sequence of SEQ ID NO:6 or a sequence at least 70% identical to said sequence.
Embodiment 12: The composition of any one of embodiments 1 to 11, wherein at least said NS1 polypeptide and said viroporin are encoded by the same polynucleotide. Embodiment 13: The composition of embodiment 12, wherein said at least one polynucleotide comprises the sequence of SEQ ID NO:5 or a sequence at least 70% identical to said sequence and/or the sequence of SEQ ID NO:6 or a sequence at least 70% identical to said sequence, preferably wherein said polynucleotide is an expression construct. Embodiment 14: The composition of any one of embodiments 1 to 13, wherein said at least one polynucleotide further comprises an expression construct encoding a VP1u polypeptide.
Embodiment 15: The composition of any one of embodiments 1 to 14, wherein said at least one polynucleotide further encodes all structural polypeptides of a parvovirus. Embodiment 16: The composition of any one of embodiments 1 to 15, wherein said expression construct encoding an NS1 polypeptide comprises a parvovirus genome. Embodiment 17: The composition of any one of embodiments 1 to 16, wherein said at least one polynucleotide comprises a parvovirus genome comprising an expression construct encoding a viroporin.
Embodiment 18: The composition of any one of embodiments 1 to 17, wherein said at least one polynucleotide is comprised in a parvovirus particle.
Embodiment 19: The composition of any one of embodiments 1 to 17, wherein said composition further comprises a transfection agent.
Embodiment 20: A composition comprising an NS1 polypeptide of a parvovirus and a viroporin, wherein the mass ratio of NS1 polypeptide to viroporin is at most 100, preferably at most 10, more preferably at most 1.
Embodiment 21: The composition according to any one of embodiments 1 to 20, wherein said composition is a pharmaceutical composition.
Embodiment 22: A composition according to any one of embodiments 1 to 20 for use as a medicine.
Embodiment 23: A composition of according to any one of embodiments 1 to 20 for use in the treatment of inappropriate cellular proliferation.
Embodiment 24: The composition for use according to embodiment 23, wherein said inappropriate cellular proliferation is cancer, in particular glioblastoma multiforme (GBM), pancreatic cancer, preferably pancreatic ductal carcinoma (PDAC), colon carcinoma, melanoma, and/or liver cancer, preferably hepatocellular cancer (HCC).
Embodiment 25: Use of the composition according to any one of embodiments 1 to 20 for the manufacture of a medicament, preferably a medicament for the treatment of cancer.
Embodiment 26: A method for treating inappropriate cellular proliferation in a subject, comprising
   a) contacting said subject with a composition according to any one of embodiments 1 to 20, and
   b) thereby treating inappropriate cellular proliferation.
Embodiment 27: A method for identifying a subject susceptible to treatment of inappropriate cellular proliferation with a composition according to any one of embodiments 1 to 20, comprising
   a) contacting a test sample of inappropriately proliferating cells of said subject with a composition according to any one of embodiments 1 to 20,
   b) determining viability of cells comprised in the sample of a) after said contacting, and
   c) thereby identifying a subject susceptible to treatment.

All references cited in this specification are herewith incorporated by reference with respect to their entire disclosure content and the disclosure content specifically mentioned in this specification.

### Figure Legends

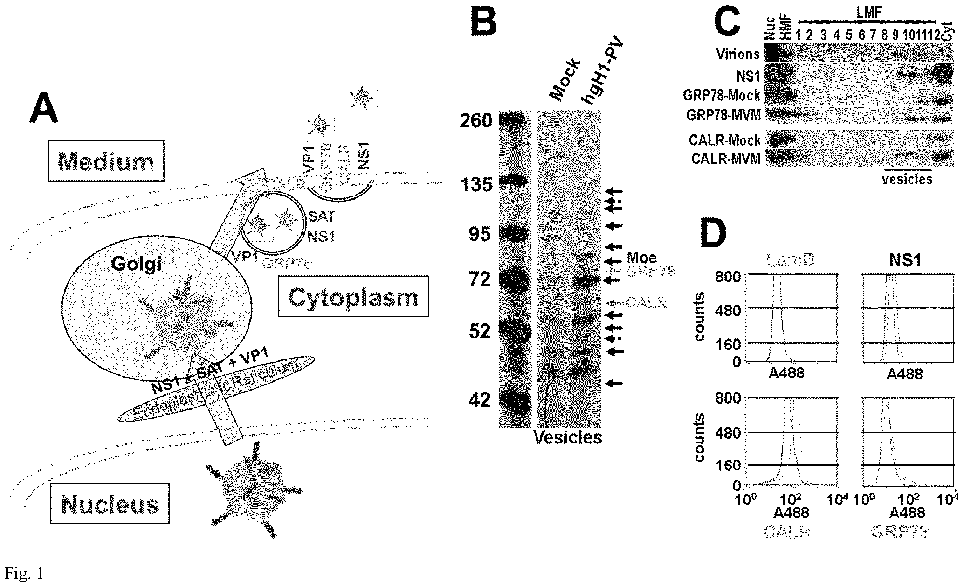

Fig. 1: **Exposure of intracellular antigens to the cell surface in PV-infected cells.** (A) Schematic presentation of the intracellular pathway of progeny particles through ER and Golgi to the Plasma membrane (Bär et al. (2013), PloS Path 9(9):e1003605). Intracellular viral (VP1, NS1, SAT) and cellular (GRP78, CALR) proteins are transported from the ER to the PM where they become exposed at the cell surface. (B) Silver stained SDS-PAGE of purified intracellular vesicles. Upon infection of glioma cells with hgH-1PV multiple cellular (e.g. moesine, GRP78, CALR) together with viral proteins become overrepresented in this compartment as compared to mock-treated cells. (C) Western Blot analyses of fractionated cellular extracts followed by purification of intracellular vesicles. Nuc, nuclear fraction; Cyt, cytoplasm, HMF, high molecular weight fraction; LMF, low molecular weight fraction. The latter was further fractionated on iodixanol gradients to obtain purified vesicles. (D) FACS-analyses of permeabilized (black) vs. non-permeabilized (grey) cells with detection of NS1, CALR, and GRP78, respectively. Lamin B served as an intracellular control. While NS1 and CALR are extensively detected at the outside of infected cells, only marginal amounts of GRP78 and no Lamin B are detected in non-permeabilized cells, although these proteins are clearly detected by the antibodies after permeabilization.

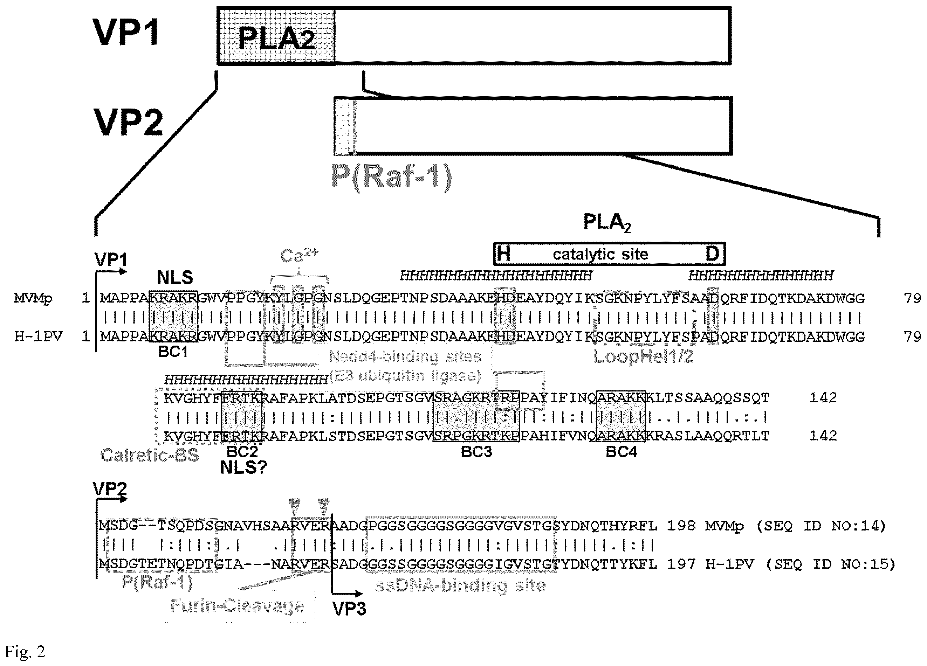

Fig. 2: **Domain-structure of VP1.** Top panel: Schematic presentation of VP1 and VP2 with the unique N-termini and the overlapping C-terminal sequences. Bottom: Sequence alignment of VPlu of MVM and H-1PV. Conserved motifs potentially serving for protein/protein interactions as well as the catalytic PLA2-site are indicated. BC1-4, basic clusters; NLS, nuclear localization signals.

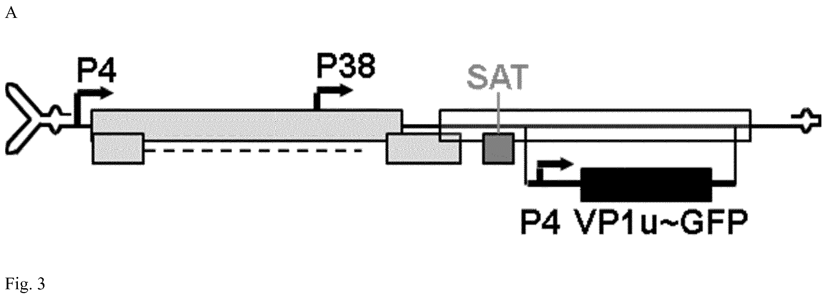

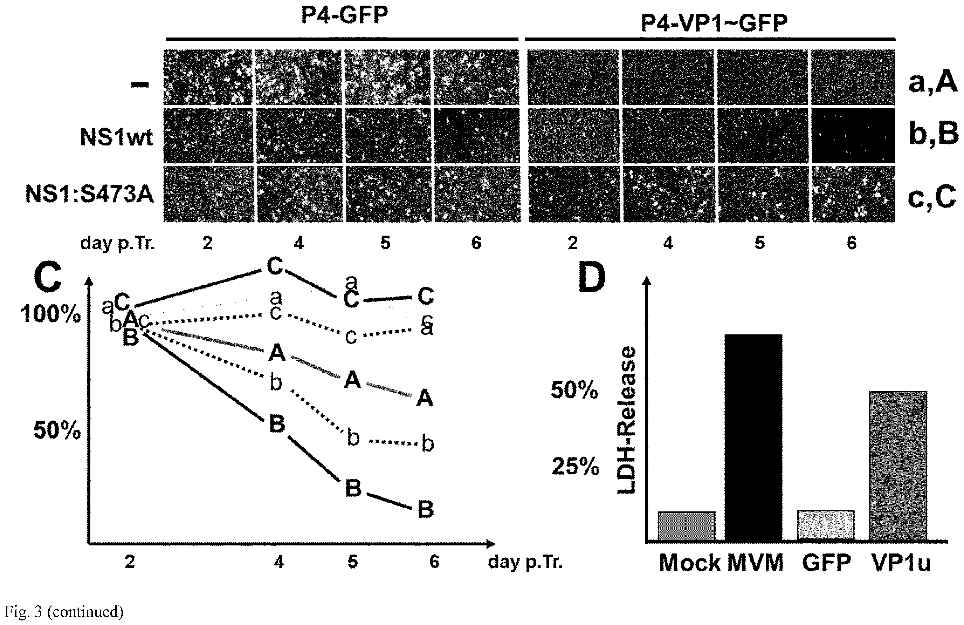

Fig. 3: **VPlu enhances NS1-toxicity and induction of cytolysis (**WO 2001/012666**).** (A) Schematic representation of a MVM vector expressing VP1u∼GFP or GFP, respectively under the control of the early P4-promoter. (B, C) Living A9 cells transfected with constructs expressing NS in combination with GFP (P4-GFP) or VP1u∼GFP, respectively are monitored over a period of 7 days for their morphology/induction of CPE (B) and the presence of viable cells (C). - , no NS proteins (green); NS1wt, wild type NS1 (red); NS1:S473A, non-toxic NS1 variant (blue); capital letters, P4-VP1u∼GFP, lower case letters, P4-GFP. (D) Lytic activity of a PV-Vector devoid of capsid expression in the presence of VP1∼GFP or GFP alone was measured after transduction of A9 at day 3 p.Transduction. MVM virus and mock-treated cells served as controls.

Fig. 4: **SAT acts as an excecuter protein for the permeabilization of membrane structures in PV-infected cells.** (A) PV genome organization. SAT is a small 56 aa polypeptided expressed through leaky scanning 4 bases after the start of VP2 in ORF2. (B) Infection of A9 cells with different multiplicities of wild-type und mutant MMVp:SATko virus. Knock-out of SAT-expression in MVMp leads to dramatic reduction of cell lysis as measured by PI-positive cells at different times of infection. (C) Transfection of 293T cells with CMV-driven plasmids expressing SAT, MycSAT, NS1 and CFP, respectively. Lytic activity is measured as mains of PI-positive cells 3 d p.Tr.

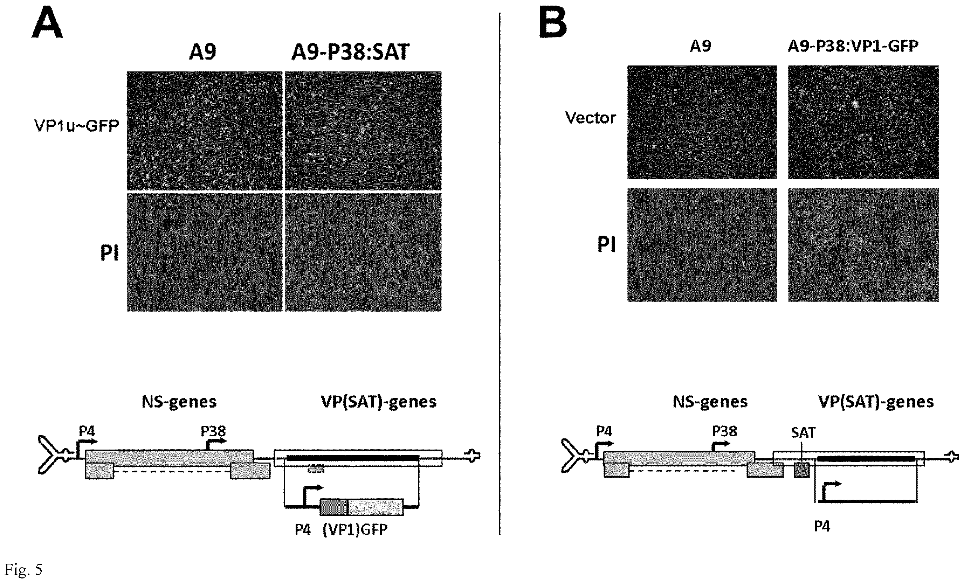

Fig. 5: **SAT and VPlu are required for efficient cytolysis of NS-driven PV-vectors.** A9 cells or derivatives were transduced with propagation incompetent MVMpl vectors at a m.o.i. of 10. 36 h p.i. lytic activity was measured by means of PI-positive cells. (A) A9 cells or A9-P38:SAT, respectively were transduced with an MVM-vector expressing NS1, NS2/3 and VP1u∼GFP driven under the control of the P4 promoter. The region of the *SAT* gene was deleted in this construct. (B) A9 cells or A9-P38:VP1u∼GFP were transduced with an MVM-vector expressing NS1, NS2/3 and SAT (pink). Large parts of the VP2 gene are deleted in this vector.

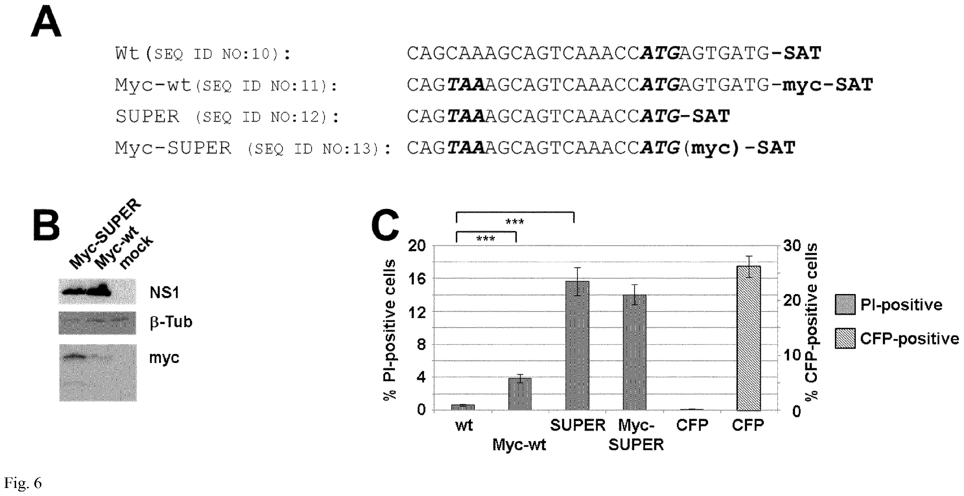

Fig. 6: **Enhanced intracellular SAT-concentration increase cytolysis in PV-derived vectors.** This can be achieved by enhanced translation initiation and stabilizing SAT through an N-terminal TAG-sequence, respectively. (A) Minimal changes in PV-vectors leading to enhanced SAT-activities in transduced cells. red TAA, stop codon introduced to abolish VP1 expression; brown ATG, start-codon for VP2; blue ATG, start codon for SAT translation; green myc, represents Myc-tag. (B) MycSAT presence in A9 cells transduced with indicated constructs as measured by western blotting. (C) Lytic activity of different Vector constructs 24 h post transfection of A9 cells. Lysed cells were detected by PI-staining. Transfection efficiency was measured by CMV-CFP.

The following Examples shall merely illustrate the invention. They shall not be construed, whatsoever, to limit the scope of the invention.

### Example 1

Following the production of viral products in cell culture over time, concomitant with the appearance of progeny virion release, exposure of intracellular components and viral regulatory proteins at the cell surface of infected cells was observed, potentially serving as damage-associated molecular patterns (DAMPs) and pathogen-associated molecular patterns (PAMPs). Among the proteins identified so far are the viral NS1 and VP1 (but not NS2), as well as the intracellular calreticuline and to a minor proportion GRP78 (Fig. 1). NS1 alone seemed to be able to stimulate cellular exocytosis (not shown), however, proved inefficient to induce cell lysis and the exposure of intracellular antigens.

Example 2: A VPlu-GFP fusion peptide was postulated earlier to modulate/stimulate NS1-toxicity (EP 1 275 658; US 10/483,234). The VPlu part (Fig. 2) of the capsid protein VP1 exerts a PLA2-activity and harbors a variety of motifs potentially serving for protein/protein as well as ssDNA interactions. The PLA2-activity was shown to be essential for the endosomal escape during the entry process through externalization from infectious virions under acidic conditions (Cotmore & Tattersall (2007), Adv Virus Res 70:183-232, Ros et al. (2017), Viruses 9(11):313). It could be shown that VPlu coupled to GFP (VPlu-GFP) strongly stimulated lytic activity of PV-vectors debilitated to produce viral capsids (EP 1 275 658; US 10/483,234). As shown in Fig. 3, VP1u∼GFP strongly stimulated NS-toxicity and was able to significantly increase LDH-release assays using MVM vectors devoid of endogenous viral capsid proteins.

### Example 3:

The constructs used to prove the involvement of VPlu in PV-induced cell lysis expressed, besides NS1, the three isoforms of NS2 (NS2p, NS2y, and NS2L), an elongated form of NS2p (termed NS3), and a novel PV protein SAT. The SAT gene is positioned four basepairs downstream of the VP2 coding region. It is expressed from reading frame 2 as an alternative translation initiation of VP2 (Fig. 4A). While analyzing the SAT protein, it was found that SAT homo-oligomerizes within cellular membranes. In addition, similarities of SAT to viroporins were found, a class of virally-expressed proteins that homo-oligomerize within membranes to make them permeable to certain molecules such as ions. During our studies we also found that SAT permeabilizes the plasma membrane thereby lysing the cell and that the lytic function of SAT is independent of other parvoviral proteins. The expression of SAT was shown to cause permeabilization of membrane structures, including the plasma membrane suggesting an important function in PV-induced oncolysis (Fig. 4C), suggesting SAT as an executer polypeptide for PV-induced oncolysis.

### Example 4:

To test the hypothesis that SAT is an executer polypeptide, we generated an MVM-derived viral vector, expressing NS1 and NS2/3 and an expression cassette to produce VP1u∼GFP under the control of the P4 promoter, however lacking the coding region of SAT. To complement for this polypeptide we produced permanent A9 cell lines harboring the SAT-gene under the NS1-inducible P38-promoter. Under these conditions the remaining SAT production in the absence is innocuous for the parental A9 cells, however, becomes induced in the presence of NS1 to achieve the proposed impact. As shown in Fig. 5A, transduction with a vector lacking the SAT-gene, but expressed VP1u∼GFP was barely able to permeabilize parental A9 cells 28 h p.Tr., however induced efficient lysis of A9:P38-SAT cells after induction of the P38 promoter, clearly demonstrating the necessity of SAT for the lytic capacity of MVM.

To assess the contribution of VPlu, we performed a similar experiment using an MVM-vector with the original SAT-gene, however, lacking a large part of the VP2 coding sequence between nts 3450 and 4200. To complement for VP1(u)-contribution, we produced stable A9 cells expressing VP1u∼GFP under the control of the P38 promoter. Interestingly, NS and SAT expression after transduction of these vectors in parental A9 cells only produced small amounts of PI-positive cells, suggesting that the limiting SAT expression under native conditions is not sufficient to induce efficient cell lysis. In contrast, these vectors efficiently induced cell lysis of A9 cells expressing VP1u∼GFP after induction through NS1, suggesting that MVM-induced permeabilization is facilitated as cooperation between NS(1), SAT and VP1u.

In summary, these observations prompts us to propose a novel Parvolysin composed of PV NS1 and SAT, where NS1 contributes to the oncotropism and in consequence regulates the viroporin SAT-induced cell lysis, optionally in cooperation with VP1u. The latter might act in (multi)protein complex as a scaffold protein through the presence of protein/protein interaction domains rather than its PLA2-activity. Notably, overexpression/stabilization of SAT can bypass the requirement for VP1u. In the proposed combination, NS1 may be a derivative from any Protoparvovirus-species, depending on the target neoplasm and SAT can probably be replaced by a related viroporin or a similar cellular polypeptide cooperating with NS1 (and/or VP1u).

Non-standard literature cited:
Angelova (2015), Front Bioeng Biotechnol 3:55;
Angelova et al. (2017), Viruses 9(12):382;
Bär et al. (2015), PLoS pathogens 11:e1004703;
Bär et al. (2013), PloS Path 9(9):e1003605;
Bretscher, Ph D Thesis; University of Heidelberg;
Cornelis et al. (2004), J Gene Med. 6 Suppl 1:S193-202;
Cotmore & Tattersall (2007), Adv Virus Res 70:183-232,
EP 1 275 658;
Feng & Doolittle (1987), J. Mol. Evolution. 25:351-360;
Geletneky et al. (2012), BMC cancer 12:99;
Geletneky et al. (2017), J Am Soc Gene Ther 25:2620;
Higgins et al. (1989), CABIOS, 5:151-153
Marchini et al. (2016), Viruses 8(1):9;
Meszaros (2017), J Virol. 91(16):e00627-17
Needleman and Wunsch (1970), J. Mol. Biol. 48:443-453;
Nuesch (2006). Regulation of non-structural protein functions by differential synthesis, modification and trafficking, p. 275-286. In Bloom MEC, S.F.; Linden, R.M.; Parrish, C.R.; and Kerr, J.R. (ed.), Parvoviruses. Hodder Arnold, London;
Nuesch & Rommelaere (2007), PNAS 104:12482-12487;
Nuesch et al. (2012), Clinical Cancer Res 18:3516-3523;
Nuesch et al. (2014), Adv Exp Med Biol 818:99-124;
Rommelaere et al. (2010), Cytokine Growth Factor Rev. 21(2-3):185;
Ros et al. (2017), Viruses 9(11):313;
Smith and Waterman (1981), Adv. Appl. Math. 2: 482-489;
US 10/483,234
WO 2001/012666
Zadori et al. ((2005), J Virol 79:13129-13138;

## Claims

1. A composition comprising at least one polynucleotide comprising (i) an expression construct encoding an NS1 polypeptide of a parvovirus and (ii) an expression construct encoding a viroporin.

2. The composition of claim 1, wherein said viroporin comprises a SAT polypeptide of a parvovirus.

3. The composition of claim 1 or 2, wherein said viroporin is a fusion polypeptide comprising a SAT polypeptide of a parvovirus and a stabilizing peptide, preferably a myc-tag, more preferably an N-terminal myc-tag.

4. The composition of any one of claims 1 to 3, wherein said viroporin comprises the amino acid sequence of SEQ ID NO:2 or a sequence at least 70% identical thereto.

5. The composition of any one of claims 1 to 4, wherein at least said NS1 polypeptide and said viroporin are encoded by the same expression construct.

6. The composition of any one of claims 1 to 6, wherein said expression construct encoding a viroporin comprises the sequence of SEQ ID NO:5 or a sequence at least 70% identical to said sequence.

7. The composition of any one of claims 1 to 6, wherein said NS1 polypeptide comprises at least the amino acids corresponding to amino acids 235 to 672 of the NS1 polypeptide of parvovirus strain MVMp (SEQ ID NO:1) or a sequence at least 70% identical thereto.

8. The composition of any one of claims 1 to 7, wherein said expression construct encoding an NS1 polypeptide comprises the sequence of SEQ ID NO:6 or a sequence at least 70% identical to said sequence.

9. The composition of any one of claims 1 to 8, wherein at least said NS1 polypeptide and said viroporin are encoded by the same polynucleotide.

10. The composition of any one of claims 1 to 9, wherein said at least one polynucleotide further comprises an expression construct encoding a VPlu polypeptide.

11. A composition comprising an NS1 polypeptide of a parvovirus and a viroporin, wherein the mass ratio of NS1 polypeptide to viroporin is at most 100, preferably at most 10, more preferably at most 1.

12. A composition according to any one of claims 1 to 11 for use as a medicine.

13. A composition of according to any one of claims 1 to 11 for use in the treatment of inappropriate cellular proliferation.

14. The composition for use according to claim 13, wherein said inappropriate cellular proliferation is cancer, in particular glioblastoma multiforme (GBM), pancreatic cancer, preferably pancreatic ductal carcinoma (PDAC), colon carcinoma, melanoma, and/or liver cancer, preferably hepatocellular cancer (HCC).

15. A method for identifying a subject susceptible to treatment of inappropriate cellular proliferation with a composition according to any one of claims 1 to 11, comprising
a) contacting a sample of inappropriately proliferating cells of said subject with a composition according to any one of claims 1 to 11,
b) determining viability of cells comprised in the sample of a) after said contacting, and
c) thereby identifying a subject susceptible to treatment.
